# EUROPEAN PATENT APPLICATION

(11) **EP 2 315 029 A1**
(43) Date of publication of application: **27.04.2011**
(21) Application number: 09173487.1
(22) Date of filing: 20.10.2009
(51) Int. Cl.: G01N 33/68

(54) **Method for the differentiation between Crohn's disease and ulcerative colitis by detecting volatile organic compounds in exhaled air.**

(71) Applicant: Universiteit Maastricht, 6211 LK Maastricht (NL); Academisch Ziekenhuis Maastricht, 6229 HX Maastricht (NL)
(72) Inventor: Dallinga, Jan Willem, 6411 VA, HEERLEN (NL); Schooten, Van, Frederik Jan, 6247 BB, GRONSVELD (NL); Berkel, Van, Joep Joseph Benjamin Nathan, 6224 CS, MAASTRICHT (NL)
(74) Representative: Habets, Winand

(57) **Abstract**

The present invention relates to the identification of markers for distinguishing between Crohn's disease and ulcerative colitis in patients with Inflammatory Bowel Disease. The uses of such markers in the differential diagnosis of inflammatory Bowel Disease and a novel method for their identification are herein described. We were able to discriminate between patients with Crohn's disease and ulcerative colitis by determining whether the exhaled air contained markers selected from that set. We also found that these markers can be used in the early diagnosis of Crohn's disease and ulcerative colitis. Moreover, these markers may be useful in the prediction of the occurrence of Crohn's disease and ulcerative colitis even when no other clinical signs are apparent yet.

## Description

### Field of the invention

The present invention relates to a method for the differentiation between Crohn's disease and ulcerative colitis in patients that have already been diagnosed with Inflammatory Bowel Disease. The method detects volatile organic compounds in exhaled air.

### Background of the invention

Inflammatory Bowel Diseases (IBD) are chronic relapsing diseases of the gastrointestinal tract and the term comprises Crohn's disease (CD) and ulcerative colitis (UC). The diagnosis of IBD and therapeutic decisions are most often based on endoscopic assessment of inflammation of mucosal tissue.

CD is characterized by chronic discontinuous transmural inflammation that may involve any portion of the gastrointestinal tract, but most commonly the terminal ileum. Fistulas or strictures may complicate CD.

UC is characterized by inflammation of the colonic mucosa, extending in a continuous manner and to a variable extent from the rectum to the proximal colon. The disease course is characterized by flairs and remissions. The differential diagnosis between CD and UC is made trough endoscopic evaluation and histology. Differentiation is necessary since both diseases behave differently and respond to different treatments.

The need for systematic evaluation of the outcome of clinical trials led to the development of disease activity indices. The Crohn's disease activity index (CDAI) is the golden standard in pharmaceutical trials in CD. (Sandborn et al., Gastroenterology. 2002;122:512-30).

The CDAI evaluates the number of liquid stools, abdominal pain, general well being, the occurrence of extra-intestinal symptoms, the need for antidiarrheal drugs, the presence of abdominal masses, hematocrit and body weight. A substantial portion of the total score is derived from the subjective parameters general well being and intensity of abdominal pain that potentially overlap with symptoms of irritable bowel disease of IBD patients in remission. Furthermore fibrotic structures can provided complaints but should be treated by dilatation or surgery. Multicenter randomized double-blind placebo controlled pharmaceutical trials performed in the last decades often show a major placebo effect. This is partly due to the natural disease course but the activity scores used also play a role. Therefore major therapeutic decisions are mostly made after endoscopic assessment of mucosal inflammation and mucosal healing became an important end-point in clinical trials.

Ileocoloscopy however is an invasive procedure which is very unpleasant for the patient with possible severe side effects. Furthermore small bowel inflammation may be missed.

As a solution to this problem, several non invasive biomarkers for mucosal inflammation in IBD have been developed. The presence of active gut inflammation in patients with IBD is associated with acute phase reaction and the migration of leucocytes to the gut. This is translated in a large number of proteins detectable in serum and stools (Vermeire et al., Gut 2006; 55:426-431). In serum the white blood cell count, C reactive protein (CRP) and erythrocyte sedimentation rate (ESR) can change during inflammation but they have a low sensitivity and specificity for mucosal inflammation. Calprotectin (Cal) is a calcium and zinc binding protein found in neutrophils and monocytes and potentially in epithelial cells. It is released in stool after cell disruption and cell death but is also actively secreted.

A meta-analysis of 30 studies including 663 patients with CD and 361 with UC and 697 with IBS and 3393 controls showed that fecal Cal was significantly higher in patients with IBD compared to controls. The sensitivity and specificity for the diagnosis of IBD was 0.95 (95% Cl0.93-0.97) and 0.91 (95%Cl0.86-0.91) (Von Roon et al., Am J Gastroenterol. 2007;102:803-13).

Hence, biomarkers may be useful as a screening tool for intestinal inflammation. However, endoscopic evaluation remains necessary for the differentiation between CD an UC.

Fecal markers are useful in the follow-up of IBD patients; however they are less sensitive for patients with only small bowel disease. Furthermore stool samples cannot always be provided. Moreover, the relation between mucosal healing and stool markers is unclear.

### Summary of the invention

We analyzed the volatile organic compounds (VOCs) in exhaled air of patients with Inflammatory Bowel Disease by using a gas chromatograph - time of flight - mass spectrometer (GC-TOF-MS). We found a set of markers that may be used for the differential diagnosis of Crohn's disease and ulcerative colitis within the group of patients diagnosed with Inflammatory Bowel Disease.

We were able to discriminate between patients with Crohn's disease and ulcerative colitis by determining whether the exhaled air contained markers selected from that set.

More in particular we found that these markers can be used in the prediction of the occurrence of Crohn's disease and ulcerative colitis even when no other clinical signs are apparent yet.

The method is also useful since it provides a new non-invasive, easy, safe, cost effective and fast diagnostic tool for the differential diagnosis of Crohn's disease and ulcerative colitis.

Hence, the invention relates to an in vitro method for distinguishing between Crohn's disease and ulcerative colitis in a patient diagnosed with Inflammatory Bowel Disease by determining the amount of at least one volatile organic compound in a sample derived from a patient diagnosed with Inflammatory Bowel Disease and comparing said amount with a predetermined reference value for said volatile organic compound, wherein a difference between the reference value and the amount of said at least one volatile organic compound is indicative of either Crohn's disease or ulcerative colitis, wherein said volatile organic compound is selected from the group consisting of 2-hexanone, 1-heptene, 2,2,6,6-tetramethylheptane, 3-(1-methylethyl)-2-cyclopenten-1-one, 1,3,5-triazine, tridecane (linear), 4-methyl-decane, 3-methylfuran, pyridine and 2,7,10-trimethyldodecane.

### Detailed description of the invention

We provide herein a panel of distinctive VOCs, which discriminate between Crohn's disease and ulcerative colitis. Based on 20 times cross validation, a correct classification of Crohn's disease and ulcerative colitis was found using at least one VOC selected from table 1.

**TABLE 1**

| *Identification of 10 VOCs capable* of *discriminating between* Crohn's disease and ulcerative colitis | | | |
|---|---|---|---|
| **VOC No** | Retention Time [min.] | Chemical formula | Identified as |
| 1 | RT 08.67 | C₆H₁₂O | 2-hexanone |
| 2 | RT 07.22 | C₇H₁₄ | 1-heptene |
| 3 | RT 15.56 | C₁₁H₂₄ | 2,2,6,6-tetramethylheptane |
| 4 | RT 12.73 | C₈H₁₂O | 3-(1-methylethyl)-2-cyclopenten-1-one |
| 5 | RT 06.76 | C₃H₃N₃ | 1,3,5-triazine |
| 6 | RT 19.05 | C₁₃H₂₈ | tridecane (linear) |
| 7 | RT 16.46 | C₁₂H₂₆ | 4-methyl-decane |
| 8 | RT 06.86 | C₅H₆O | 3-methylfuran |
| 9 | RT 09.00 | C₅H₅N | pyridine |
| 10 | RT 22.22 | C₁₅H₃₂ | 2,7,10-trimethyldodecane |

The compounds listed in table 1 can be distinguished on the basis of their chemical formula only, or in a number of cases by the combination of their chemical formula and the retention time of the compound on the specific column used (see examples section). Under the circumstances used, the retention time of toluene was 9.15 minutes. The skilled person will appreciate that the retention times as referred herein are approximate values subject to the usual experimental variance.

Hence, the invention relates to an in vitro method of distinguishing between Crohn's disease and ulcerative colitis in a patient by determining the amount of at least one volatile organic compound in a sample derived from a patient suspected of having Inflammatory Bowel Disease and comparing said amount with a predetermined reference value for said volatile organic compound, wherein a difference between the rference value and the amount of said at least one volatile organic compound is indicative of Inflammatory Bowel Disease, wherein said volatile organic compound is selected from the group consisting of 2-hexanone, 1-heptene, 2,2,6,6-tetramethylheptane, 3-(1-methylethyl)-2-cyclopenten-1-one, 1,3,5-triazine, tridecane (linear), 4-methyl-decane, 3-methylfuran, pyridine and 2,7,10-trimethyldodecane.

Said sample derived from a patient is preferably a sample of exhaled air from the patient, it may also be a sample wherein the volatile organic compounds in the exhaled air are concentrated, collected and/or immobilized, such as an active carbon solid phase or an affinity matrix.

A method according to the invention makes a contribution to the art as a whole, in that it provides a method with a sensitivity of more than 50% while the specificity is more than 60%.

Methods to detect VOCs in exhaled air as such are known in the art. The examples provide guidance for the skilled person to perform an analysis according to the invention. Reference values may be obtained by determining the average value of these VOCs in the exhaled air of IBD patients.

VOCs may be detected in several ways known in the art. This may be done by a device capable of measuring more than one VOC at the same time such as a mass spectrometer or by a device specifically suitable for measuring only one VOC at a time.

When a single VOC is to be used, VOC No 1, i.e. a hydrocarbon with molecular formula of C₆H₁₂O and retention time of 8.67 min is preferred. This may be advantageous in an assay capable of detecting only a single VOC at a time such as an electronic nose.

As is shown in table 2, the sensitivity, specificity, and therewith the percentage of correct classification increases with increasing number of VOCs selected from table 1. Hence, the invention also relates to a method as described above wherein at least two volatile organic compounds are detected, such as three, four, five or six VOCs. The method may even be further improved by determining the relative amounts of seven, eight, or even nine VOCs. Most preferred is a method as described above wherein all ten VOCs from table 1 are determined in a method according to the invention.

The percentage of correct classification increases when more than one VOC selected from table 1 are used in a method according to the invention. The highest sensitivity and specificity could be obtained when a method according to the invention was performed with all 10 VOCs from table 1 combined.

A preferred embodiment of the method according to the invention comprises the use of VOC No 1 together with a VOC selected from the group consisting of VOC No 2, No 3, No 4, No 5, No 6, No 7, No 8, No 9 and No. 10. Preferable VOC number 2 is combined with VOC No 1.

It was shown that a method according to the invention resulted in a sensitivity of 97.4% and a specificity of 90.7% when 2 VOCs were used according to table 1. Further performance characteristics are shown in table 2.

**Table 2**

| Number of VOCs | sensitivity(%) | specificity (%) |
|---|---|---|
| 10 | 97.4 | 90.7 |
| 9 | 86.8 | 83.7 |
| 8 | 76.3 | 88.4 |
| 7 | 76.3 | 88.4 |
| 6 | 73.7 | 83.7 |
| 5 | 65.8 | 79.1 |
| 4 | 50.0 | 51.2 |
| 3 | 89.5 | 51.2 |
| 2 | 97.4 | 90.7 |
| 1 | 86.8 | 83.7 |

VOC can easily be determined by collection of exhaled breath and quantitative analysis by thermal desorption-gas chromatography time-of-flight mass spectrometry (GC-TOF-MS) [Van Berkel JJBN, et al., J Chrom B 2008;861:101-7].

In Medicine and Life Sciences often Receiver operating characteristic (ROC) curves are used as a measure for the strength of an analysis. A ROC curve is a graphical representation of the sensitivity versus 1-specificity for a binary classifier system, at varying discrimination thresholds. It is well usable for the results of a discriminant analysis with exactly one discriminant function, as is the case in the experiments we performed here. The area under the curve value (AUC) is a measure for the reliability of the method; a value of 1 is found when the method perfectly discriminates between positive and negative patients, a value of 0.5 represents a method that does not discriminate at all. Values between 0.9 and 1 are considered excellent, values between 0.8 and 0.9 are considered good.

Area under curve (AUC)-values were calculated for discriminant functions based on 4, 3 and 2 components, respectively. The results show that the method discriminates well between healthy individuals and patients with IBD for every component in table 1. The AUCs for the VOCs selected from table 1 are: 0.796, 0.847 and 0.846 for a method employing 2, 3 or 4 VOC's selected from table 1 respectively.

When comparing the abundance of a particular VOC with the predetermined reference value, it is referred to table 3 for the reference values obtained in the experimental set-up as described in the below examples. It will be clear to the skilled person that each different method for determining VOCs (electronic nose, time-of-flight detector or otherwise) requires its own determination of reference values. Table 3 indicates that VOCs 1, 6, 7, 8 and 10 are higher in Ulcerative Colitis patients as compared to patients with Crohn's disease, whereas VOCs 2, 3, 4, 5 and 9 are higher in Crohn's disease.

**Table 3: Relative abundance of VOCs described herein in the exhaled air from patients with UC versus patients with CD in percentage of total x 100.**

| **VOC #** | **UC(43)** | **CD (38)** |
|---|---|---|
| 1 | 1,98 | 0,29 |
| 2 | 14,19 | 21,61 |
| 3 | 1,05 | 6,08 |
| 4 | 0,28 | 0,61 |
| 5 | 3,58 | 16,82 |
| 6 | 7,74 | 0,61 |
| 7 | 6,05 | 3,50 |
| 8 | 2,14 | 0,34 |
| 9 | 0,07 | 0,18 |
| 10 | 4,79 | 4,37 |

### Examples

### Example 1:Patient population

Exhaled air was collected from 38 patients with CD, 43 patients with UC and 38 healthy controls. UC and CD patient populations contained patients with active disease as well as patients in remission. Remission was defined as a CDAI below 150 or a SCCAI below 2.5 for CD and UC respectively. Smokers and non smokers were equally divided among groups.

### Example 2: Sample collection

Patients and healthy controls were asked to exhale into resistance free plastic Tedlar bags (5L) and the content of the bag was transported under standardized conditions onto stainless steel two-bed sorption tubes, filled with carbograph 1TD/Carbopack X (Markes International, Llantrisant, Wales, UK) that trapped the VOCs and stored until analysis

### Example 3: VOC Analysis

Analysis of the samples was performed as previously described [Van Berkel JJBN, et al., J Chrom B 2008;861:101-7]. In brief; the volatile compounds trapped on the sorption tubes by thermal desorption were released, using the Markes International Ultra-Unity automated thermal desorption equipment (Markes International, Llantrisant, Wales, UK). The gaseous mixture of released compounds was then split; 90% of the sample was recollected on a second identical sample tube and stored for an optional second analysis. 10% of the sample was loaded onto a cold (5 °C) sorption trap, from which it was injected into a gas chromatograph (Trace GC, Thermo Fischer Scientific, Austin, Texas, USA) and analysed by time-of-flight mass spectrometry (Tempus Plus, Thermo Fischer Scientific, Austin, Texas, USA).

For the GC-MS measurements the following conditions were used: column: Restek RTX-5ms, 30m x 0.25 mm ID, coated with 1.0 um HP-5 phase; helium was used as the carrier gas at a flow rate of 1.5 ml / min. The temperature of the gas chromatograph was programmed as follows: 40 °C for 5 min. then increased by 10 °C / min until 270 °C, at which temperature it was maintained for 5 min. The mass spectrometer was set at a scan range of 35-350 AMU and scanned 5 times per second. The complete analytical procedure, including sampling, storage and instrumental analysis was tested for reproducibility. Instrumental reproducibility was determined by the analysis of identical exhaled air samples that were obtained by emptying a filled bag over y-shaped connector onto two absorption tubes. The two absorption tubes were subsequently analyzed by GC-TOF-MS. This experiment was repeated 6 times.

The quantification of the similarity was done by means of calculation of a distance measure (dot product rule). This distance measure is based on the similarity of the entire raw chromatogram. Distance measure calculation of all complementary files resulted in a distance measure ranging from 0.96 to 0.99. (A value of '1' denotes identical samples, the lower the value the lesser the degree of similarity). Intra-individual variability was examined by repeated sampling of exhaled air from 10 subjects for 5 consecutive days and comparing the results per subject from day to day. Inter-individual variability was examined by sampling 10 subjects and comparing the data from subject to subject. Again the similarity between several chromatograms was quantified using a distance measure as mentioned above. The intra-individual variability ranged from 0.80 to 0.99 and is far smaller than the inter-individual variability, ranging from 0.16 to 0.98

### Example 4: Data analysis

The GC-MS chromatograms of the breath samples of the subjects involved in this study were recorded and retention times were normalized by calculating retention indices, relative to toluene and lining up using easily recognizable component peaks, to correct for chromatographic drifting. Parts of the chromatograms that occurred at a retention index < 0.15 and at a retention index > 2.8 were removed from the chromatograms, because of unreliable data from these parts, due to noisy mass spectra at the beginning of the chromatograms and column bleeding at the end of each run.

The remaining data were transformed to Excel files, containing almost 6000 different chromatographic peaks, determined by retention time and mass spectrum and combined with a relative intensity. In our approach the measured mass spectra were compared to one another at the same retention time, rather than to library spectra.

In this way the resemblance of the original spectra determined the decision whether peaks at the same retention time represent the same component or not intensities below the detection limit were set at 0%. The detailed descriptions of the data handling procedures can be found elsewhere [Van Berkel JJBN, et al., J Chrom B 2008;861:101-7.].

Before data analysis was executed, all peaks that occurred in less than 8% of the samples (meaning less than ten times in this study) were removed. This resulted in a final data matrix, containing 945 peaks and 120 subjects. This matrix was normalized by adjusting the sum of all peak intensities in each subject at 100%. This way the influence of the infrequently appearing peaks could be diminished. The resulting data were analysed by a stepwise discriminant analysis, using SPSS (SPSS13.0 for windows, SPSS Inc. Chicago, Illinois, USA).

The discriminant analyses were performed using a twentyfold crossover approach. In this method all but 5 percent of the chromatograms (in this study 5 percent equals 6 samples) are used to construct the discriminant function, which is subsequently used to predict to which group the ones left out belong. This is repeated 20 times, until all samples have once been classified.

## Claims

1. In vitro method for distinguishing between Crohn's disease and ulcerative colitis in a patient diagnosed with Inflammatory Bowel Disease by determining the amount of at least one volatile organic compound in a sample derived from a patient diagnosed with Inflammatory Bowel Disease and comparing said amount with a predetermined reference value for said volatile organic compound, wherein a difference between the reference value and the amount of said at least one volatile organic compound is indicative of either Crohn's disease or ulcerative colitis, wherein said volatile organic compound is selected from the group consisting of 2-hexanone, 1-heptene, 2,2,6,6-tetramethylheptane, 3-(1-methylethyl)-2-cyclopenten-1-one, 1,3,5-triazine, tridecane (linear), 4-methyl-decane, 3-methylfuran, pyridine and 2,7,10-trimethyldodecane.

2. Method according to claim 1 wherein said method comprises the step of determining at least the amount of 2-hexanone in said sample.

3. Method according to claim 2 wherein said method consists of the step of determining the amount of 2-hexanone in said sample

4. Method according to claims 1 or 2 wherein at least 2 volatile organic compounds are detected.

5. Method according to claim 4 wherein at least 3 volatile organic compounds are detected.

6. Method according to claim 5 wherein at least 4 volatile organic compounds are detected.

7. Method according to claim 6 wherein at least 5 volatile organic compounds are detected.

8. Method according to claim 7 wherein at least 6 volatile organic compounds are detected.

9. Method according to claim 8 wherein at least 7 volatile organic compounds are detected.

10. Method according to claim 9 wherein at least 8 volatile organic compounds are detected.

11. Method according to claim 10 wherein at least 9 volatile organic compounds are detected.

12. Method according to claim 11 wherein at least 10 volatile organic compounds are detected.
